Europäisches Patentamt

(19) European Patent Office

Office européen des brevets

(11) Publication number: **0 011 868**
A1

(12) **EUROPEAN PATENT APPLICATION**

(21) Application number: **79104788.9**

(22) Date of filing: **30.11.79**

(51) Int. Cl.³: **A 61 K 9/00**
**A 61 K 31/195**

(30) Priority: **01.12.78 US 965642**

(43) Date of publication of application:
**11.06.80 Bulletin 80/12**

(84) Designated Contracting States:
**AT BE CH DE FR GB IT LU NL SE**

(71) Applicant: **MERCK & CO. INC.**
**126, East Lincoln Avenue P.O. Box 2000**
**Rahway New Jersey 07065(US)**

(72) Inventor: **Dempski, Robert E.**
**1629 Arran Way**
**Dresher, Pa. 19025(US)**

(72) Inventor: **O'Neill, Joseph L.**
**2211 Oakwyn Road**
**Lafayette Hill, Pa. 19444(US)**

(74) Representative: **Abitz, Walter, Dr.-Ing. et al,**
**Abitz, Morf, Gritschneder P.O. Box 86 01 09**
**D-8000 München 86(DE)**

(54) Methyldopa composition.

(57) An aqueous suspension containing methyl-dopa and sucrose is disclosed. This composition is an oral dosage form for treating hypertension that is bioavailable.

EP 0 011 868 A1

- 1 -                              16246

METHYLDOPA COMPOSITION

BACKGROUND OF THE INVENTION

The present invention concerns a liquid suspension containing methyldopa useful for oral treatment of hypertension.

Methyldopa (L-3-(3,4-dihydroxyphenyl)-2-methylalanine) is a known antihypertensive agent. It is generally administered orally for treating a hypertensive patient. The dosage form commonly used is a tablet. In some instances e.g. with children or the very elderly, administration of methyldopa in a palatable, liquid dosage form would be advantageous provided that it had a bioavailability at least equivalent to that of the tablet form.

Such a liquid pharmaceutical composition containing methyldopa has been discovered.

SUMMARY OF THE INVENTION

A liquid pharmaceutical composition for treating hypertension containing methyldopa and sucrose.

DESCRIPTION OF THE PREFERRED EMBODIMENTS

An embodiment of the present invention is a pharmaceutical composition for treating hypertension in humans comprising an aqueous suspension containing methyldopa and sucrose.

This suspension can contain from about 15 mg to about 100 mg of methyldopa per milliliter (ml) of aqueous suspension. It will preferably contain from 25 mg. to 100 mg. of methyldopa, and most preferably about 50 mg. of methyldopa, per ml. of said suspension.

The suspension must also contain from about 1 mg. to about 24 mg. of sucrose per mg. of methyldopa. Preferably, the suspension will contain from about 1 mg. to about 12 mg. of sucrose per mg. of methyldopa; more preferably the weight ratio of methyldopa:sucrose is about 1:2 to about 1:10 and most preferably about 1:10.

In addition to the essential ingredients, methyldopa and sucrose, the composition of the present invention can contain other ingredients conventionally used to prepare a pharmaceutically useful suspension. Such ingredients include antioxidants, e.g. sodium bisulfite, disodium edetate and citric acid, thickening/suspending agents, e.g . xanthan gum and Veegum K, flavorings, preservatives, coloring agents and the like. Conventional procedures and equipment are used to prepare the present suspensions.

An outstanding characteristic of the present suspension is that the bioavailability of the methyldopa is equal to or greater than methyldopa administered in conventional tablet form.

When sorbitol, which is very commonly used to prepare pharmaceutically useful suspensions, is substituted for the sucrose in the present suspension, the bioavailability of the methyldopa is substantially less than methyldopa administered as a tablet. This is indeed suprising since sorbitol is reported to enhance the bioavailability of other pharmaceutically active compounds such as vitamins, (C & E, News, p. 59-60, February 24, 1958).

The bioavailability of methyldopa was determined in vivo by measuring the concentration of methyldopa and conjugated methyldopa in the plasma and urine of humans subjects to whom 500 mg of methyldopa were administered in standard tablet form and in suspension form. These measurements showed the bioavailability of methyldopa in the various dosage forms to be as follows:

Suspension containing sucrose
= or tablet    suspension containing
sorbitol.

Thus, the present suspension dosage form is bioequivalent to methyldopa in conventional tablet form.

Following are formulations illustrating the compositions of the present invention:

| Ingredient | I | | II | | III | | IV | |
|---|---|---|---|---|---|---|---|---|
| Methyldopa USP Milled (Anhydrous) | 50.0 | mg. | 50.0 | mg. | 100 | mg. | 100 | mg. |
| Microcrystalline Cellulose and Sodium Carboxymethylcellulose NF (Avicel RC591 NF) | 10.0 | mg. | 10.0 | mg. | 10.0 | mg. | 10.0 | mg. |
| Disodium Edetate USP | 0.50 | mg. | 0.50 | mg. | 0.50 | mg. | 0.50 | mg. |
| Sodium Bisulfite USP | 2.00 | mg. | 2.00 | mg. | 2.00 | mg. | 2.00 | mg. |
| Citric Acid Monohydrate | 1.00 | mg. | 1.00 | mg. | 1.00 | mg. | 1.00 | mg. |
| Sugar USP Med. Gran. | 500 | mg. | 200 | mg. | 500 | mg. | 200 | mg. |
| Glycerin USP | 10.0 | mg. | 10.0 | mg. | 10.0 | mg. | 10.0 | mg. |
| Ethyl Alcohol USP 95% | 0.011 | ml. | 0.011 | ml. | 0.011 | ml. | 0.011 | ml. |
| Polysorbate 80 USP | 0.20 | mg. | 0.20 | mg. | 0.20 | mg. | 0.20 | mg. |
| Benzoic Acid | 1.00 | mg. | 1.00 | mg. | 1.00 | mg. | 1.00 | mg. |
| Orange Flavor Comp. Fritzsche Dodge & Olcott 16242 | 0.0004 | ml. | 0.0004 | ml. | 0.0004 | ml. | 0.0004 | ml. |
| Pineapple Art. Flavor Fritzsche Dodge & Olcott 05194 | 0.0002 | ml. | 0.0002 | ml. | 0.0002 | ml. | 0.0002 | ml. |
| Water Purified USP q.s. | 1.0 | ml. | 1.0 | ml. | 1.0 | ml. | 1.0 | ml. |

- 4 -

0011868

- 5 -                          16246

Claims to the invention follow.

WHAT IS CLAIMED IS:

1.  A pharmaceutical composition for the oral treatment of hypertension comprising an aqueous suspension containing methyldopa and sucrose.

2.  The composition of Claim 1 containing from about 1 to about 12 mg of sucrose per mg of methyldopa.

3.  The composition of Claim 1 containing from about 2 to about 10 mg of sucrose per mg of methyldopa.

4.  The composition of Claim 3 wherein said suspension contains about 50 mg of methyldopa per ml of said aqueous suspension.

5.  The composition of Claim 3 wherein said suspension contains about 500 mg. of sucrose per ml of said aqueous suspension.

6.  The composition of Claim 3 containing about 50 mg. of methyldopa and about 500 mg. of sucrose per ml. of said aqueous suspension.

7.  The composition of Claim 1 which is characterized by being bioequivalent to methyldopa in tablet form.

Claims for Austria

1. A process for preparing an aqueous suspension containing from about 1 to about 12 parts by weight of sucrose per part by weight of methyldopa which comprises

1.) preparing a hydrated aqueous suspension containing about 10 parts by weight of microcrystalline cellulose/sodium carboxymethylcellulose,

2.) dissolving, with stirring, in the 1.) suspension from about 96 to about 500 parts by weight of sucrose,

3.) adding, with stirring, about 8 to about 500 parts by weight of milled methyl-dopa to the mixture from 2.),

4.) dissolving, with stirring, about 10 parts by weight of glycerin in the 3.) mixture,

5.) dissolving in sufficient ethanol about 1 part by weight of benzoic acid, about 20 parts by weight of polysorbate 80, a flavor effective amount of flavoring agent and adding this ethanol solution to the 4.) mixture, with stirring,

6.) adding, with stirring, to the 5.) mixture an aqueous solution containing about 50 parts by weight of disodium edetate,

7.) adding, with stirring, to the 6.) mixture an aqueous solution containing about 0.2 to 2 parts by weight of sodium bisulfite,

8.) adding, with stirring, to the 7.) mixture an aqueous solution containing about 0.1 - 1 parts by weight of citric acid,

and

9.) finally, adding sufficient water to the 8.) mixture so that the final suspension will provide from about 15 mg/ml to about 100 mg/ml of methyldopa.

2. The process of claim 1 wherein ingredient concentrates are such that said final suspension will provide from about 25 mg/ml to about 100 mg/ml methyldopa.

3. The process of claim 1 wherein said final suspension will provide from about 50 mg/ml to about 100 mg/ml of methyldopa.

4. The process of claim 1 wherein said final suspension will provide about 100 mg/ml of methyldopa.

5. The process of claim 1 wherein said final suspension will provide about 50 mg/ml of methyldopa.

| Category | Citation of document with indication, where appropriate, of relevant passages | Relevant to claim |
|---|---|---|
| X | CHEMICAL ABSTRACTS, vol. 83, no. 20, 17th November 1975, page 270, column 2, no. 168409c Columbus, Ohio, U.S.A. D.W. NEWTON et al.: "Extemporaneous preparation of methyldopa in two sirup vehicles" & AM. J. HOSP. PHARM. 1975, 32(8), 817-21 (Eng.) * Abstract * -- | 1-7 |
| | US - A - 3 526 698 (GERALD P. POL-LI) * Column 2, lines 3-68; column 3, line 42 - column 4, line 20; column 4, lines 21-29; claim 1 * -- | 1,7 |
| A | GB - A - 957 586 (MERCK & CO., INC.) * Claims 2,7-9 * -- | 1 |
| A | US - A - 3 976 782 (WALFRED S. SAARI) * Column 3, line 64 - column 4, line 23; column 6, example 6 * ---- | 1 |

**DOCUMENTS CONSIDERED TO BE RELEVANT**

CLASSIFICATION OF THE APPLICATION (Int. Cl. )

A 61 K 9/00
31/195

TECHNICAL FIELDS SEARCHED (Int.Cl )

A 61 K 9/00
31/195

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 13-03-1980 | BRINKMANN |

EPO Form 1503.1 06.78